(19) Europäisches Patentamt
European Patent Office
Office européen des brevets

(11) EP 0 474 013 B1

(12) EUROPÄISCHE PATENTSCHRIFT

(45) Veröffentlichungstag und Bekanntmachung des Hinweises auf die Patenterteilung:
**28.02.1996 Patentblatt 1996/09**

(51) Int. Cl.[6]: **C07D 501/12**, C07D 501/36

(21) Anmeldenummer: **91113868.3**

(22) Anmeldetag: **19.08.1991**

(54) **Verfahren zur Herstellung von kristalliner TACS**
Process for the preparation of crystalline TACS
Procédé de préparation de TACS crystallin

(84) Benannte Vertragsstaaten:
**AT BE CH DE DK ES FR GB IT LI NL SE**

(30) Priorität: **23.08.1990 DE 4026630**

(43) Veröffentlichungstag der Anmeldung:
**11.03.1992 Patentblatt 1992/11**

(73) Patentinhaber: **HOECHST AKTIENGESELLSCHAFT**
**D-65926 Frankfurt am Main (DE)**

(72) Erfinder:
- **Trödel, Reinhard**
**W-6203 Hochheim a.M. (DE)**
- **Wieduwilt, Manfred, Dr.**
**W-6000 Frankfurt a.M. (DE)**

(56) Entgegenhaltungen:
**DE-A- 3 542 644**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 474 013 B1

Printed by Rank Xerox (UK) Business Services
2.9.9/3.4

## Beschreibung

Die vorliegende Erfindung betrifft ein Kristallisationsverfahren zur Isolierung von 7-β-Amino-3-(5-carboxymethyl-4-methyl-1,3-thiazol-2-yl-thiomethyl)-ceph-3-em-4-carbonsäure (TACS) der Formel

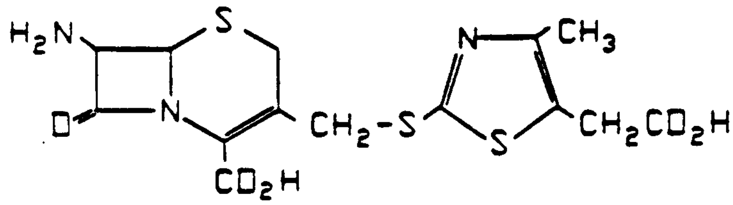

TACS

TACS ist das zentrale Zwischenprodukt bei der Herstellung des Human-Antibiotikums Cefodizim,

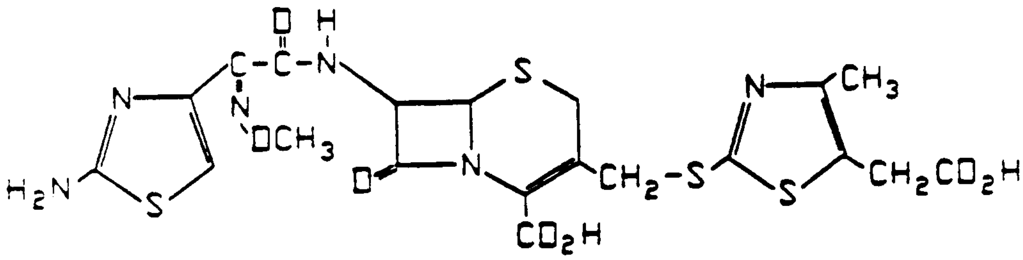

Cefodizim

das durch Acylierung der TACS erhalten wird. Cefodizim wird als steriles Dinatriumsalz gegen bakterielle Infektionen eingesetzt.

Die Herstellung der TACS durch Umsetzung von 7-Aminocephalosporansäure (7-ACS)

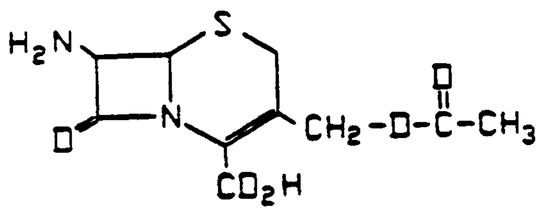

7-ACS

und 2-Mercapto-4-methyl-5-carboxymethyl-1,3-thiazol (MMTS)

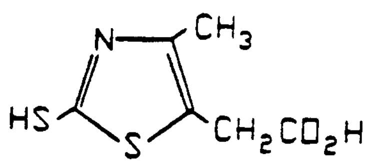

MMTS

in Wasser in Gegenwart einer Base (Natriumbicarbonat) und Ausfällen durch Zugabe einer Säure (2n Salzsäure, pH 2,0) wird in U.S. 4 278 793 beschrieben. Dabei wird durch eine Austauschreaktion in der Seitenkette der 7-ACS der

MMTS-Rest eingeführt und so die TACS gebildet. Zweckmäßig ist es, zur Ausfällung der TACS nicht nur Säure, sondern auch noch eine Fällhilfe, vorzugsweise einen Alkohol, hinzuzugeben. Das so erhaltene Kristallisat wird dann in üblicher Weise geschleudert, auf der Schleuder gewaschen, isoliert und getrocknet.

Dieses im Prinzip literaturbekannte Verfahren, bei dem die Ausfällung der TACS durch Zugabe einer Säure erfolgt, wurde im Labor- und Technikumsmaßstab und in seiner großtechnischen Anwendungsmöglichkeit überprüft, wobei auch noch ein üblicher chromatographischer Reinigungsschritt eingeschaltet wurde.

Im Labor- und Technikumsmaßstab kann die Fällung der TACS so durchgeführt werden, daß die über eine Adsorptionschromatographiesäule gereinigte Lösung der herstellungsbedingt als Salz, vorzugsweise Natriumsalz vorliegenden TACS zunächst mit einem Fällhilfsmittel, z.B. einem Alkohol, vorzugsweise n-Butanol, versetzt wird. Gibt man nun eine Säure, bevorzugt eine anorganische Säure, vorzugsweise Schwefelsäure, hinzu und stellt durch Rühren eine homogene Phase her, so bleibt zunächst die an TACS übersättigte Lösung klar, bis nach einiger Zeit die Kristallisation einsetzt.

Das so gewonnene Kristallisat ist zwar im Maßstab von wenigen Kilogramm, nicht jedoch im großtechnischen Rahmen in akzeptabler Zeit ohne Qualitätsverlust mit Hilfe einer Zentrifuge von seiner Mutterlauge zu separieren, zu waschen und zu isolieren. Als Ausweg bleibt bei dieser Kristallisationsweise nur, die Ansatzgröße drastisch zu reduzieren, um infolge der dann erheblich kleineren Ansatzgröße auf der Zentrifuge eine so geringe Schichtdichte an Kristallisat vorliegen zu haben, daß der gesamte Isolierungsprozeß ohne Qualitätseinbuße bei akzeptabler Zeitdauer möglich ist.

Im Rahmen der großtechnischen Verfahrensentwicklung zur Herstellung der TACS wurde eine Methode ausgearbeitet, TACS aus der Lösung als Kristallisat auszufällen. Diese Methode führt jedoch - wie unten gezeigt wird - bei einer großtechnischen Produktionsmenge dazu, daß die Isolierzeit für das Kristallisat auf einer Zentrifuge dermaßen lange dauert, daß bereits eine deutliche Produktschädigung in Bezug auf Farbe, Gehalt und Ausbeute eintritt.

Die Fällung erfolgte hierbei so, daß nach Austausch mit MMTS in der 3'-Position von 7-ACS zur TACS die TACS-Reaktionslösuing über eine mit Adsorptionsharz, vorzugsweise HP 20, gefüllte Chromatographiesäule gereinigt wurde. Die produktenthaltende Eluatfraktion wurde mit einem Fällhilfsmittel, insbesondere einem Alkohol, vorzugsweise n-Butanol, versetzt. Innerhalb einer bestimmten Zeit, vorzugsweise von etwa 30 Minuten, wurde durch Zugabe einer Säure, vorzugsweise einer anorganischen Säure, insbesondere von ca. 15 %iger Schwefelsäure, ein definierter pH-Wert von vorzugsweise etwa 5,5 eingestellt. Bei dieser Zugabe oder kurz danach begann die TACS zu kristallisieren.

Es wurde kurzzeitig, vorzugsweise etwa 15 Minuten, nachgerührt und dann durch weitere Zugabe der Säure ein definierter End-pH-Wert von vorzugsweise etwa 4,0 eingestellt. Man rührte einige Zeit, vorzugsweise etwa 1 Stunde, nach und gab die Suspension auf eine Zentrifuge. Die Mutterlauge wurde bei mäßiger Drehzahl, vorzugsweise 300 - 400 Upm, abgeschleudert und der verbleibende Kristallkuchen mit Waschflüssigkeit, vorzugsweise einem Alkohol, insbesondere Isopropanol, gewaschen. Nach dem Trockenschleudern bei gleicher Drehzahl, vorzugsweise etwa 400 Upm, wurde der Feststoff isoliert und getrocknet.

Diese Vorgänge dauerten im Maßstab von etwa 100 kg Produkt/Ansatz (dies entspricht einer Schleuderfüllung) mehr als 30 Stunden. Dabei wurde eine zunehmende Verfärbung des Feststoffs beobachtet, so daß die getrocknete TACS die gesetzten Qualitätskriterien nicht mehr erfüllte. Der Ansatz war damit für eine Weiterverarbeitung nicht brauchbar.

Es bestand also ein Bedürfnis, ein Kristallisationsverfahren zu finden, das es erlaubt, im großtechnischen Maßstab TACS zu kristallisieren, zu zentrifugieren, zu waschen und zu isolieren, ohne daß Qualitätsverluste während der dazu notwendigen Zeit auftreten.

Gegenstand der vorliegenden Erfindung ist daher ein Verfahren zur Herstellung von kristalliner TACS

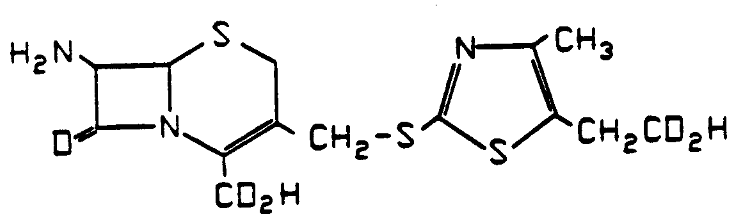

TACS

durch Umsetzung von 7-ACS

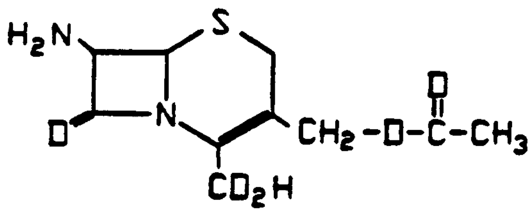

7-ACS

und MMTS

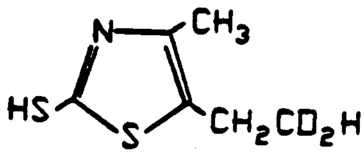

MMTS

in Gegenwart einer Base und anschließende Ausfällung der TACS durch Zugabe einer Säure, das dadurch gekennzeichnet ist, daß die Ausfällung der TACS durch Zugabe einer TACS-Lösung zu einer Teilmenge der Säure, die auch noch ein Fällhilfsmittel enthalten kann, erfolgt, wobei die Ausfällung durch weitere Säurezugabe bis zum End-pH-Wert von 3,0 - 4,5 oder bei Konstanthaltung des End-pH-Wertes durch weitere Zugabe von TACS-Lösung und Säure vervollständigt wird.

Durch die vorliegende Erfindung wird die Art der Kristallisation durch Agglomeration von feinkristallinem Material so verändert ("Inverse Kristallisation"), daß die TACS anschließend in großtechnischem Maßstab in akzeptabler Zeit ohne Qualitätseinbuße auf einer Zentrifuge geschleudert und gewaschen, sowie isoliert werden kann. Dieses Material hält die gesetzten Qualitätskriterien und ist für die Weiterverarbeitung sehr gut geeignet.

Das Fällhilfsmittel, insbesondere ein niedrigmolekularer Alkohol, vorzugsweise n-Butanol oder Amylalkohol, wird zusammen mit einer Teilmenge, vorzugsweise etwa 40 - 50 %, der zur Überführung des TACS-Salzes, vorzugsweise des Natriumsalzes, in die freie Carbonsäure berechneten Gesamtmenge an Säure vorgelegt. Die Berechnung erfolgt anhand des vorher jeweils zu bestimmenden Gehalts des Eluats an TACS. Als Säuren kommen anorganische Säuren, wie z.B. Salzsäure oder vorzugsweise Schwefelsäure, insbesondere etwa 15 %ige Schwefelsäure, oder organische Säuren, wie z.B. Ameisensäure oder Essigsäure in Betracht.

Innerhalb einer kurzen Zeit, vorzugsweise von etwa 15 Minuten, gibt man die über ein Adsorptionsharz, vorzugsweise HP 20, gewonnenen, zuvor gesammelten Eluatfraktionen unter Rühren zu. Dabei setzt sofortige Kristallisation ein und der pH-Wert steigt auf einen Wert von vorzugsweise etwa 5,3 bis 5,4. Nach kurzzeitigem, vorzugsweise etwa 5-minütigem Rühren wird durch weitere Zugabe von Säure, vorzugsweise in etwa 10 Minuten, der End-pH-Wert von etwa 3,0 - 4,5, insbesondere 3,8-4,2, vorzugsweise 4,0, eingestellt. Man rührt kurze Zeit, vorzugsweise etwa 5 Minuten, nach und läßt längere Zeit, vorzugsweise etwa 1,5 Stunden, ohne Rühren absitzen. Dann ist es möglich, einen größeren Teil, etwa 1/3 bis 1/2 der Mutterlauge, als klaren Überstand über der Suspension abzuhebern. Dieser abgeheberte Teil wird, da er nur wenig feinsten Feststoff enthält, nicht über die Zentrifuge gegeben. Die verbleibende, das Kristallisat enthaltende Suspension gibt man nun auf eine Zentrifuge. Die Mutterlauge wird bei mäßiger Drehzahl, vorzugweise etwa 300 - 400 Upm, abgeschleudert und der verbleibende Kristallkuchen mit Waschflüssigkeit, vorzugsweise einem Alkohol, vorzugsweise Isopropanol, gewaschen. Nach dem Trockenschleudern bei gleicher Drehzahl, vorzugweise etwa 400 Upm, wird der Feststoff isoliert und getrocknet.

Diese Vorgänge dauern im Maßstab von etwa 100 kg Produkt/Ansatz (dies entspricht einer Schleuderfüllung) weniger als 6 Stunden. Dabei wird keine Verfärbung des Feststoffs beobachtet. Die getrocknete TACS erfüllt die gesetzten Qualitätskriterien und ist damit für die Weiterverarbeitung zum Cefodizim gut geeignet.

Das erfindungsgemäße Verfahren kann auch mit geringfügigen Modifikationen durchgeführt werden. So ist es möglich, auch ganz auf das Fällhilfsmittel zu verzichten und nur die Teilmenge der Säure vorzulegen, oder auch die anorganische Säure durch eine organische Säure, wie zum Beispiel Ameisensäure, beispielsweise 18 %ige Ameisensäure oder Essigsäure, zu ersetzen. Die inverse Kristallisationsmethode kann auch so abgewandelt werden, daß nach Vorlegen einer Teilmenge der Säure und gegebenenfalls eines Fällhilfsmittels, Eluat zugegeben und nach Erreichen und unter weiterer Konstanthaltung des oben definierten End-pH-Wertes gleichzeitig noch weiteres Eluat und die restliche Säure hinzugefügt werden.

Das erfindungsgemäße Verfahren mit seinen Varianten führt dazu, daß die TACS in großtechnischem Maßstab in dem dafür erforderlichen Zeitraum ohne Qualitätsverlust isoliert werden kann. Damit ist die großtechnische Herstellung von TACS im Maßstab von etwa 100 kg Produkt/Ansatz überhaupt erst möglich geworden. Nachdem die bisher übliche Zugabe von Säure zur Ausfällung der TACS schlechte Ergebnisse brachte, war es nicht vorauszusehen, daß die erfindungsgemäß wesentliche Vorlage einer Teilmenge der Säure zu derartig überraschenden Resultaten führen würde.

Die folgenden Ausführungsbeispiele dienen der weiteren Erläuterung des erfindungsgemäßen Verfahrens, ohne es darauf zu beschränken.

Beispiel 1:

Herstellung des TACS-haltigen Eluats

In einem 2000 ml-Vierhalskolben werden unter Stickstoffüberlagerung

| | |
|---|---|
| 486 ml | E-Wasser vorgelegt und unter Rühren |
| 22,8 g | (0,120 Mol) Mercaptomethylthiazolessigsäure (MMTS) eingetragen. Durch Zutropfen von ca. |
| 14,1 g | (10,2 ml) Natronlauge (33%ig) wird die MMTS bei pH 6,3 bis 6,6 innerhalb von etwa 10 Minuten gelöst. Die Lösung wird anschließend auf 70°C erwärmt. |

Zwischenzeitlich werden in einem 250 ml-Becherglas

| | |
|---|---|
| 28,61 g | (0,100 Mol) 7-Aminocephalosporansäure (7-ACS) mit |
| 97,9 ml | E-Wasser zu einer homogenen Suspension verrührt. Die 7-ACS-Suspension wird innerhalb von etwa 55 Minuten mit einer Dosierpumpe zur MMTS-Lösung dosiert. Dabei wird die Innentemperatur bei 70°C gehalten. Gleichzeitig mit der Dosierung wird der pH-Wert der Reaktionslösung durch Dosierung von insgesamt ca. |
| 240 g | ( 225 ml) Natriumhydrogencatbonatlösung (8%ig) bei pH 6,3 bis 6,4 gehalten. |

Nach beendeter Dosierung wird das 7-ACS-Gefäß mit

| | |
|---|---|
| 22 ml | E-Wasser ausgespült und das Spülwasser zur Reaktionslösung gegeben. Zur vollständigen Umsetzung wird das Reaktionsgemisch eine Stunde bei 70°C nachgerührt. |

Reinigung:

Nach beendeter Reaktion wird die Lösung auf 30°C abgekühlt und mit 3 BV/h über eine Säule mit

| | |
|---|---|
| 300 ml | Adsorptionsharz HP 20 gepumpt. |

Die ersten dreihundert Milliliter des Eluats (Vorlauf) werden verworfen. Das restliche Eluat wird gesammelt. Nach beendeter Dosierung der Reaktionslösung wird die Säule mit

| | |
|---|---|
| 700 ml | E-Wasser gespült (v = 3 BV/h ) und das Wascheluat mit den Produkteluat vereinigt. |

Fällung:

Das gesammelte Eluat wird in einem 2000 ml-Vierhalskolben vorgelegt und mit

| | |
|---|---|
| 72,5 g | (89,5 ml) n-Butanol versetzt. Unter langsamen Rühren wird bei 25° bis 30°C mit insgesamt ca. |
| 83 ml | Schwefelsäure (15%ig) innerhalb etwa 30 Minuten zuerst auf pH 5,5 gestellt (35 ml), etwa 15 Minuten nachgerührt, dann weiter innerhalb von etwa 30 Minuten auf pH 4,0 gestellt (48 ml) und etwa eine Stunde bei 20°C bis 25°C nachgerührt. Danach wird abgesaugt, mit |
| 250 ml | E-Wasser und |
| 250 ml | Propanol-2 gewaschen und über Nacht bei Raumtemperatur im Vakuum getrocknet. |
| | Auswaage: 30,59 g |

Regeneration der Adsorptionsharzsäule:

In einem beheizbaren Gefäß werden

| | |
|---|---|
| 250 ml | Natronlauge (1 N) und |
| 250 ml | Propanol-2 vorgelegt, auf 70°C erwärmt und mit 1 BV/h über die Adsorptionssäule gepumpt. Anschließend wird mit |
| 500 ml | E-Wasser und |
| 1000 ml | Essigsaure (1%ig) gewaschen, bis im Säulenablauf ein pH-Wert von 5 bis 7 erreicht wird. Mit ca. |
| 250 ml | E-Wasser wird die Säule zurückgespült und über Nacht absitzen gelassen. |

Beispiel 2:

In einem 2000 ml-Vierhalskolben werden

| | |
|---|---|
| 44,0ml | Schwefelsäure (15%ig) und |
| 69,5ml | n-Butanol vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren |
| 41,0ml | Schwefelsäure(15%ig) pH 4,0 eingestellt und die entstandene Kristallsuspension eine Stunde bei 20° bis 25°C nachgerührt. Der Feststoff wird abfiltriert, mit |
| 250 ml | E-Wasser und |
| 250 ml | Propanol-2 gewaschen und über Nacht bei Raumtemperatur im Vakuum getrocknet. |
| | Auswaage: 30,90 g |

Beispiel 3:

In einem 2000 ml-Vierhalskolben werden

| | |
|---|---|
| 39,5ml | Schwefelsäure (15%ig) vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren |
| 40,1ml | Schwefelsäure (15%ig) pH 4,0 eingestellt und die entstandene Kristallsuspension etwa eine Stunde bei 20° bis 25°C nachgerührt. Der Feststoff wird abfiltriert, mit |
| 250 ml | E-Wasser und |
| 250 ml | Propanol-2 gewaschen und über Nacht bei Raumtemperatur im Vakuum getrocknet. |
| | Auswaage: 29,41 g |

Beispiel 4:

In einem 2000 ml-Vierhalskolben werden

| | |
|---|---|
| 43,0ml | Ameisensäure (18%ig) vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren |
| 47,1ml | Ameisensäure pH 4,0 eingestellt und die entstandene Kristallsuspension etwa eine Stunde bei 20° bis 25°C nachgerührt. Der Feststoff wird abfiltriert, mit |
| 250 ml | E-Wasser und |
| 250 ml | Propanol-2 gewaschen und über Nacht bei Raumtemperatur im Vakuum getrocknet. |
| | Auswaage: 30,40 g |

Beispiel 5:

In einem 2000 ml-Vierhalskolben werden

| | |
|---|---|
| 43,0ml | Ameisensäure (18%ig) und |
| 69,5ml | n-Butanol vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren |
| 50,0ml | Ameisensäure (18%ig) pH 4,0 eingestellt und die entstandene Kristallsuspension etwa eine Stunde bei 20° bis 25°C nachgerührt. Der Feststoff wird abfiltriert, mit |
| 250 ml | E-Wasser und |

250 ml Propanol-2 gewaschen und über Nacht bei Raumtemperatur im Vakuum getrocknet.
Auswaage: 29,87 g

Beispiel 6:

In einem 2000 ml-Vierhalskolben werden

37,0ml Essigsäure (99,8%ig) vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren
38,7ml Essigsäure (99,8%ig) pH 4,0 eingestellt und die entstandene Kristallsuspension etwa eine Stunde bei 20° bis 25°C nachgerührt. Der Feststoff wird abfiltriert, mit
250 ml E-Wasser und
250 ml Propanol-2 gewaschen und über Nacht bei Raumtemperatur in Vakuum getrocknet.
Auswaage: 30,30 g

Beispiel 7:

In einem 2000 ml-Vierhalskolben werden

37,0ml Essigsäure (99,8%ig) und
69,5ml n-Butanol vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren
54,0ml Essigsäure (99,8%ig) pH 4,0 eingestellt und die entstandene Kristallsuspension etwa eine Stunde bei 20° bis 25°C nachgerührt. Der Feststoff wird abfiltriert, mit
250 ml E-Wasser und
250 ml Propanol-2 gewaschen und über Nacht bei Raumtemperatur im Vakuum getrocknet.
Auswaage: 30,47 g

Beispiel 8:

In einem 2000 ml-Vierhalskolben werden

40,0ml Schwefelsäure (15%ig) und
69,5ml Amylalkohol (Gemisch aus 70% n- und 30% iso-Amylalkohol) vorgelegt. Unter Rühren wird innerhalb von etwa 5 Minuten analog Beispiel 1 bereitetes TACS-Säuleneluat zudosiert. Nach einer Nachrührzeit von etwa 5 Minuten wird mit weiteren
42,0ml Schwefelsäure (15%ig) pH 4,0 eingestellt und die entstandene Kristallsuspension etwa eine Stunde bei 20° bis 25°C nachgerührt. Der Feststoff wird abfiltriert, mit
250 ml E-Wasser und
250 ml Propanol-2 gewaschen und über Nacht bei Raumtemperatur im Vakuum getrocknet.
Auswaage: 30,60 g

Beispiel 9:

In einem 2000 ml-Vierhalskolben werden

35,0ml Schwefelsäure (15%ig) vorgelegt. Unter Rühren wird innerhalb von etwa 5 Minuten analog Beispiel 1 bereitetes TACS-Säuleneluat zudosiert. Sobald pH 4,0 erreicht ist, werden gleichzeitig
43,0ml Schwefelsäure (15%ig) so zudosiert, daß pH 4,0 gehalten wird. Die entstandene Kristallsuspension etwa eine Stunde bei 20° bis 25°C nachgerührt. Der Feststoff wird abfiltriert, mit
250 ml E-Wasser und
250 ml Propanol-2 gewaschen und über Nacht bei Raumtemperatur im Vakuum getrocknet.
Auswaage: 30,81 g

Beispiel 10:

In einem 2000 ml-Vierhalskolben werden

35,0ml Schwefelsäure (15%ig) und

| | |
|---|---|
| 69,5ml | n-Butanol vorgelegt. Unter Rühren wird innerhalb von etwa 5 Minuten analog Beispiel 1 bereitetes TACS-Säuleneluat zudosiert. Sobald pH 4,0 erreicht ist, werden gleichzeitig |
| 50,0ml | Schwefelsäure (15%ig) so zudosiert, daß pH 4,0 gehalten wird. Die entstandene Kristallsuspension etwa eine Stunde bei 20° bis 25°C nachgerührt. Der Feststoff wird abfiltriert, mit |
| 250 ml | E-Wasser und |
| 250 ml | Propanol-2 gewaschen und über Nacht bei Raumtemperatur im Vakuum getrocknet. |
| | Auswaage: 30,98 g |

**Patentansprüche**

1. Verfahren zur Herstellung von kristalliner TACS

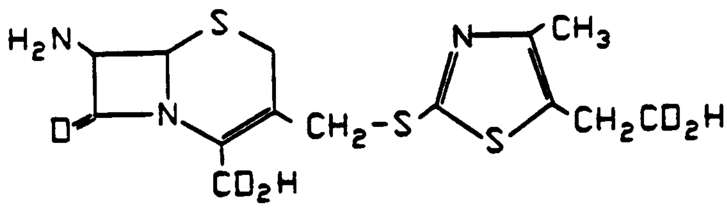

TACS

durch Umsetzung von 7-ACS

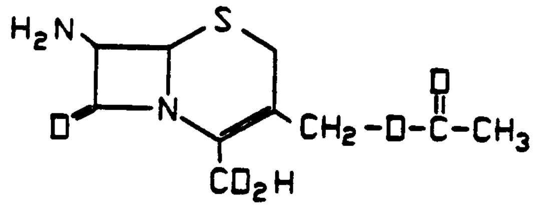

7-ACS

und MMTS

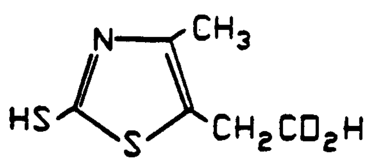

MMTS

in Gegenwart einer Base und anschließende Ausfällung der TACS durch Zugabe einer Säure, dadurch gekennzeichnet, daß die Ausfällung der TACS durch Zugabe einer TACS-Lösung zu einer Teilmenge der Säure, die auch noch ein Fällhilfsmittel enthalten kann, erfolgt, wobei die Ausfällung durch weitere Säurezugabe bis zum End-pH-Wert von 3,0 - 4,5 oder bei Konstanthaltung des End-pH-Wertes durch weitere Zugabe von TACS-Lösung und Säure vervollständigt wird.

2. Verfahren nach Anspruch 1, dadurch gekennzeichnet, daß die TACS-Lösung ein über ein Adsorptionsharz gewonnenes Eluat darstellt.

3. Verwendung der nach den Ansprüchen 1 und 2 erhaltenen TACS zur Herstellung von Cefodizim.

## Claims

1. A process for the preparation of crystalline TACA

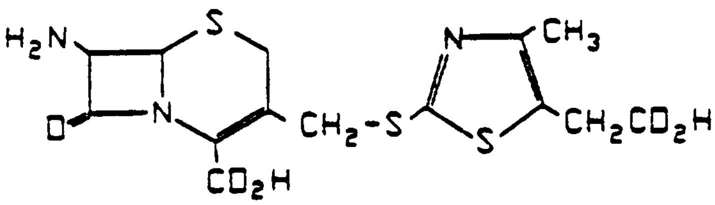

TACA

by reaction of 7-ACA

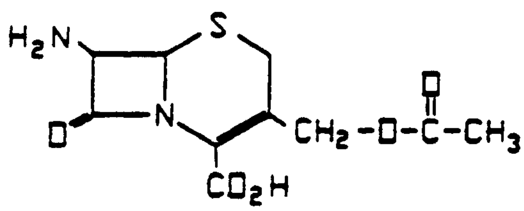

7-ACA

and MMTA

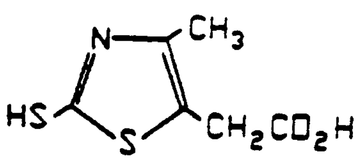

MMTA

in the presence of a base and subsequent precipitation of the TACA by addition of an acid, which comprises precipitating the TACA by addition of a TACA solution to a partial amount of the acid, which can also contain a precipitation auxiliary, the precipitation being completed by further addition of acid to a final pH of 3.0 - 4.5 or, if the final pH is kept constant, by further addition of TACA solution and acid.

2. The process as claimed in claim 1, wherein the TACA solution is an eluate obtained by means of an adsorption resin.

3. The use of the TACA obtained as claimed in claims 1 and 2 for the preparation of cefodizime.

## Revendications

1. Procédé de préparation de TACS cristallin

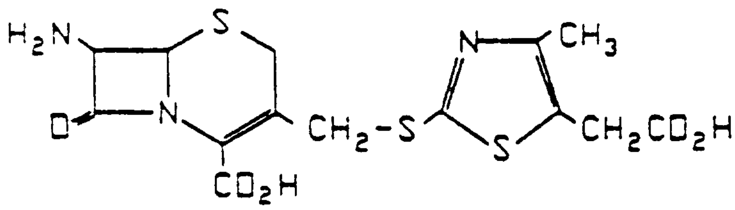

TACS

par réaction de 7-ACS

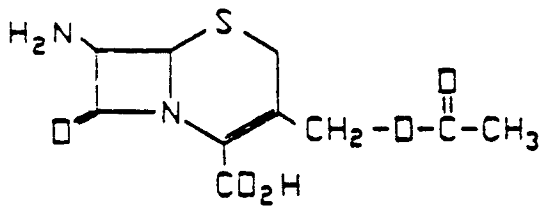

7-ACS

et de MMTS

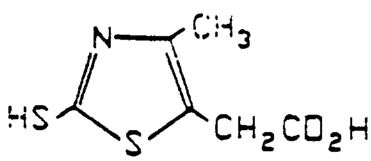

MMTS

en présence d'une base, suivie d'une précipitation du TACS sous l'ajout d'un acide, caractérisé en ce que la précipitation du TACS a lieu en ajoutant une solution de TACS à une quantité partielle de l'acide, qui peut également contenir en outre un adjuvant de précipitation, la précipitation étant complétée par une addition supplémentaire d'acide jusqu'à un pH final de 3,0 à 4,5 ou par le maintien du pH final à une valeur constante par ajout supplémentaire de solution de TACS et d'acide.

2. Procédé selon la revendication 1, caractérisé en ce que la solution de TACS est un éluat obtenu sur une résine d'adsorption.

3. Utilisation de TACS obtenu selon les revendications 1 et 2 pour la fabrication de céfodizime.